# EUROPEAN PATENT SPECIFICATION

(11) **EP 3 698 766 B1**
(45) Date of publication and mention of the grant of the patent: **09.06.2021**
(21) Application number: 19158444.0
(22) Date of filing: 21.02.2019
(51) Int. Cl.: A61H 1/02, A61H 3/00, A61B 5/00, A61B 5/11, B25J 9/00

(54) **HUMAN ASSISTIVE DEVICE COMPRISING A SENSING MODULE CAPABLE OF REDUCING NOISE**
HILFSVORRICHTUNG FÜR MENSCHEN MIT EINEM SENSORMODUL MIT FÄHIGKEIT ZUR RAUSCHVERMINDERUNG
DISPOSITIF D'ASSISTANCE HUMAINE COMPRENANT UN MODULE DE DÉTECTION CAPABLE DE RÉDUIRE LE BRUIT

(43) Date of publication of application: 26.08.2020
(73) Proprietor: Lin, Zhong-Jheng, Tainan City 709 (TW); Kuo, Yue-Jyuan, Tainan City 709 (TW)
(72) Inventor: Lin, Zhong-Jheng, Tainan City 709 (TW)
(74) Representative: Straus, Alexander

(56) References cited:
- EP-A1- 2 923 683
- US-A1- 2011 164 949
- US-A1- 2011 251 533
- US-A1- 2016 015 589
- US-B2- 7 862 524

## Description

### Field of the Invention

The present invention relates to a human assistive device, and more particularly, to a human assistive device comprising a sensing module capable of reducing noise generated by muscle swelling and skin friction during human body movement.

### Background of the Invention

The purpose of a human assistive device is to use power output from the device to aid a human body in performing tasks such as patient rehabilitation or moving heavy loads that exceed the capacity of the human body. Typically, a human assistive device has pressure sensors that contact the human body, and the deformation of the pressure sensors during human body movement generates corresponding sensor signals. The signals then result in the power output apparatus of the human assistive device to produce corresponding movement to assist the human body. However, the conventional pressure sensors contact the human body, and human muscle swelling during movement could cause friction against the pressure sensors, leading to unnecessary noise and affecting the power output from the power output apparatus of the human assistive device.

In European patent number EP 2923683 A1, a two-degree-of-freedom planar rehabilitating, monitoring and/or evaluating device is disclosed. The device comprises a first guiding mechanism (2), a second guiding mechanism (3), substantially parallel to the first guiding mechanism (2), a third guiding mechanism (4), substantially perpendicular to the first and second guiding mechanisms (2, 3), and an end-effector (5), wherein the third guiding mechanism (4) is connected to the first and second guiding mechanisms (2,3), and wherein the end-effector (5) is connected to the third guiding mechanism (4) . In US patent application US 2011/0251533 A1, a wearable robot system for rehabilitation training of the upper limbs is disclosed. The robot links move correspondingly to the motion of the upper limbs while decreasing the volume of a rehabilitation and assistance device based on a robot for rehabilitation training of the upper limbs without interfering with the human body by creating a plurality of robot motion paths. In US patent application 2016/0015589 A1, a supporting frame and a motion assistance apparatus including the same is disclosed. The supporting frame includes a first frame with a hinge connecting portion, a second frame configured to slidingly move with respect to the first frame, and an assistance force sensing portion on at least one of the first frame and the second frame. In US patent application 2011/0164949A1, a compact exoskeleton arm support device is disclosed. The compact exoskeleton arm support device compensates for gravity by using at least five joints. Among the at least five joints, two joints may be driven by actuators, and the remaining joints may be driven by user force. In US patent 7,862,524, an exoskeleton interface apparatus that parallels human arm motion is disclosed. A plurality of transmission modules is co-located on adjoining linkages to power the joints, and force is exchanged with the human at the handgrip and elbow brace

In detail, above mentioned US 2011/164949 A1 discloses a human assistive device comprising a sensing module capable of reducing noise generated by muscle swelling and skin friction during human body movement, the human assistive device comprising a torso portion, a first exoskeleton member, and a second exoskeleton member, one end of the first exoskeleton member being pivoted to the torso portion, the second exoskeleton member being pivoted to another end of the first exoskeleton member, the sensing module being positioned on at least one of the first exoskeleton member and the second exoskeleton member, the sensing module comprising: a body attaching member configured to be attached to a human limb; a base module fixed onto at least one of the first exoskeleton member and the second exoskeleton member and comprising a carrying platform fixed onto the second exoskeleton member, and a cup member disposed on the carrying platform, the cup member encasing a cup-shaped hollow space, the cup-shaped hollow space having an opening; the sensing module further comprising a transmission member and a sensor, wherein the body attaching member drives the transmission member to activate the sensor.

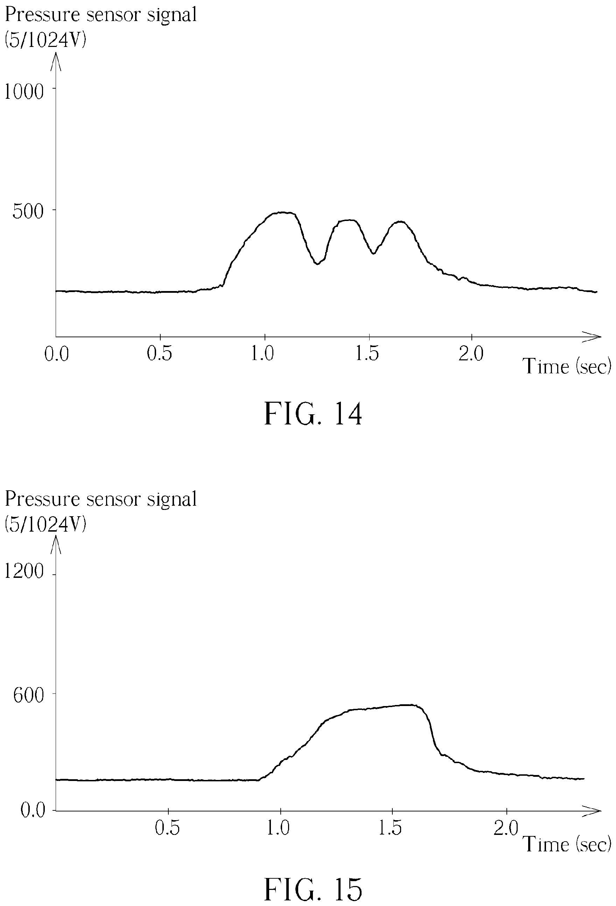

In the prior art, the pressure sensors configured on the human assistive device may contact the human body directly. Since limb movement results in muscle swelling and friction, the waveforms from the pressure sensors will contain noise that affects the power output of the power output apparatus. The pressure sensors are typically used in combination with a noise filter. Please refer to FIG. 14. FIG. 14 is a diagram illustrating the experimental signal of the pressure sensor configured on the human assistive device in the prior art. When a forearm swings relative to an upper arm, an upper arm muscle swells and causes the pressure sensor on the upper arm to produce unstable signals shown in FIG. 14.

### Summary of the Invention

With this in mind, the present invention aims at providing a human assistive device comprising a sensing module capable of reducing noise generated by muscle swelling and skin friction during human body movement.

This is achieved by a human assistive device according to claim 1. The dependent claims pertain to corresponding further developments and improvements.

As will be seen more clearly from the detailed description following below, the claimed human assistive device comprising a sensing module includes a torso portion, a first exoskeleton member, and a second exoskeleton member. One end of the first exoskeleton member is pivoted to the torso portion. The second exoskeleton member is pivoted to another end of the first exoskeleton member. The sensing module is positioned on at least one of the first exoskeleton member and the second exoskeleton member. The sensing module includes a body attaching member, a base module, a transmission member, a first sensor, a mounting base, an activating part, an initial position-constraining member, and a second sensor. The body attaching member is configured to be attached to a human limb. The base module is fixed onto at least one of the first exoskeleton member and the second exoskeleton member, and the base module comprises a carrying platform fixed onto the second exoskeleton member along with a cup member disposed on the carrying platform. The cup member encases a cup-shaped hollow space, and the cup-shaped hollow space has an opening. The transmission member is disposed in the cup-shaped hollow space and comprises a first connecting end and a second connecting end. The mounting base is disposed on an inner wall of the cup member, the first sensor is disposed on the mounting base, and the activating part is fixed onto the transmission member. The activating part is configured to activate the first sensor. The initial position-constraining member is disposed inside the cup-shaped hollow space. The second sensor is disposed on the mounting base and corresponds (in location) to the first sensor, being separate from the body attaching member. The first connecting end of the transmission member is connected to the body attaching member via the opening. The body attaching member drives the transmission member to activate the first sensor to generate a first signal when the body attaching member is driven along a first direction by the human limb. The initial position-constraining member holds the second connecting end and allows a gap to be present between the activating part and the mounting base when the transmission member is in an initial status. The body attaching member drives the activating part to activate the second sensor to generate a second signal when the body attaching member is driven by the human limb along a second direction opposite to the first direction.

The sensing module of the present invention is attached to the human limb by the body attaching member, and the sensor(s) of the sensing module may be disposed on a cup member, and the cup member is separate from the body attaching member. Furthermore, the sensing module of the present invention connects the transmission member to the body attaching member, so that the transmission member is driven to activate the sensor (s) to generate the signal (s) when the body attaching member is driven by the human limb during movement. The sensor (s) of the present invention can be free from the effects of muscle swelling and skin friction during human limb movement due to the separation of the sensor (s) from the human limb. Subsequently, an actuating module can drive the exoskeleton member to move more precisely. Furthermore, a rotating bearing disclosed herein is able to reduce the friction on the sensor (s) during the swinging motion of the arm, thereby extending the lifetime of the pressure sensor (s) while simultaneously decreasing the associated noise.

### Brief Description of the Drawings

In the following, the invention is further illustrated by way of example, taking reference to the accompanying drawings thereof, wherein the examples of Figs. 1-8 are not covered by the claims:
FIG. 1 is a diagram illustrating a human assistive device;
FIG. 2 is a diagram illustrating a first exoskeleton member, a second exoskeleton member, and a sensing module;
FIG. 3 is a diagram illustrating the first exoskeleton member, the second exoskeleton member, and the sensing module in another view;
FIG. 4 is a diagram illustrating the sensing module;
FIG. 5 is a partially exploded diagram illustrating the sensing module;
FIG. 6 is a diagram illustrating the sensing module in another view;
FIG. 7 is a front view illustrating the sensing module;
FIG. 8 is a functional block diagram of the human assistive device;
FIG. 9 is a partial diagram illustrating the human assistive device according to an embodiment of the present invention;
FIG. 10 is a diagram illustrating the sensing module according to an embodiment of the present invention;
FIG. 11 is a partially exploded diagram illustrating the sensing module according to an embodiment of the present invention;
FIG. 12 is an exploded diagram illustrating the sensing module according to an embodiment of the present invention;
FIG. 13 is a cross-section diagram of the sensing module along section line X-X as shown in FIG. 10;
FIG. 14 is a plot demonstrating signals from a conventional pressure sensor when the pressure sensor is configured on the human assistive device;
FIG. 15 is a plot demonstrating signals of the first sensor on the forearm (i.e., the human limb) when the forearm moves relative to the upper arm along a retracted direction according to another embodiment of the present invention; and
FIG. 16 is a diagram illustrating the sensing module according to the another embodiment of the present invention.

### Detailed Description

In the following detailed description of the embodiments, reference is made to the accompanying drawings which form a part hereof, and in which is shown by way of illustration specific embodiments in which the invention may be practiced. In this regard, directional terminology, such as "top," "bottom," etc., is used with reference to the orientation of the figure being described. The components of the present invention can be positioned in a number of different orientations. As such, the directional terminology is used for purposes of illustration and is in no way limiting.

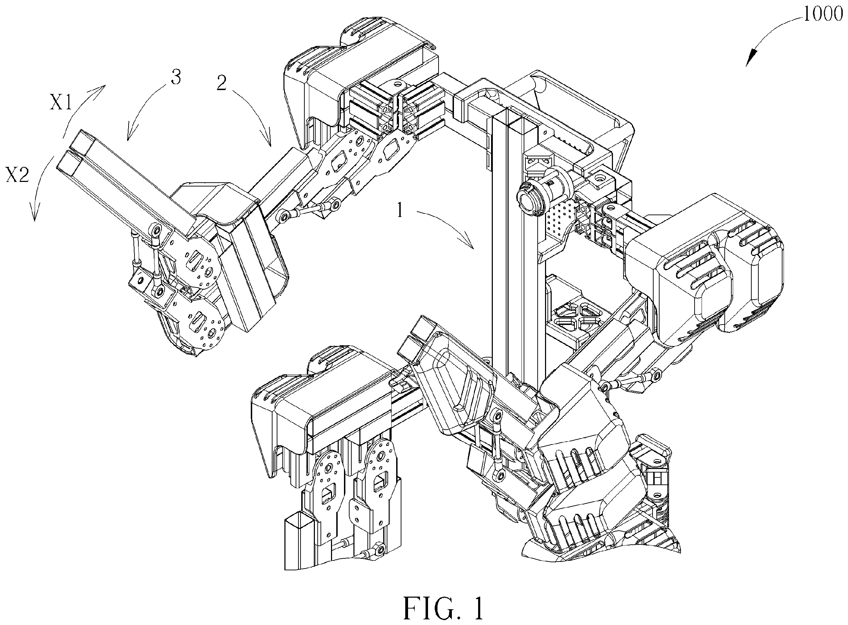

FIG. 1 is a diagram illustrating a human assistive device 1000. As shown in FIG. 1, the human assistive device 1000 includes a torso portion 1, a first exoskeleton member 2, a second exoskeleton member 3, and a sensing module 4. One end of the first exoskeleton member 2 is pivoted to the torso portion 1, another end of the first exoskeleton member 2 is pivoted to the second exoskeleton member 3. In the present embodiment, the human assistive device 1000 can be used to aid the movement of a human limb when a human body is injured, or the human assistive device 1000 can be configured as an exoskeleton device to assist the human body to move loads that exceed the capacity of the human body.

In the present embodiment, the first exoskeleton member 2 can be attached to the upper arm of the human body, and the second exoskeleton member 3 can be attached to the forearm of the human body. Furthermore, the sensing module 4 is configured on the first exoskeleton member 2 and the second exoskeleton member 3, but the present invention is not limited thereto. For example, the sensing module 4 can be configured only on the first exoskeleton member 2 or only on the second exoskeleton member 3. That is, a mechanical design in which the sensing module 4 is configured on at least one of the first exoskeleton member 2 and the second exoskeleton member 3 is within the scope of the present invention.

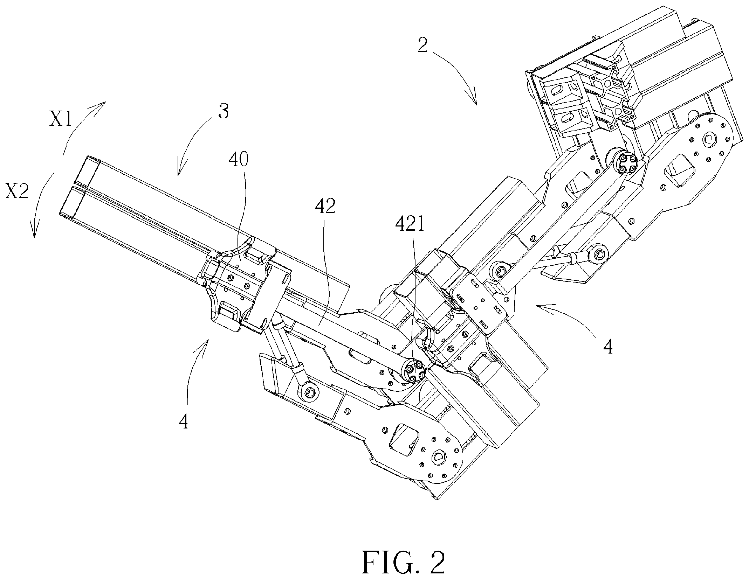

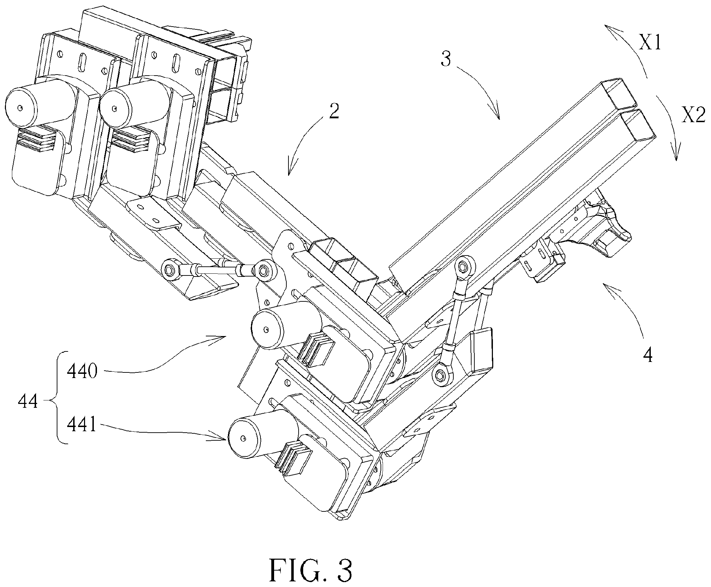

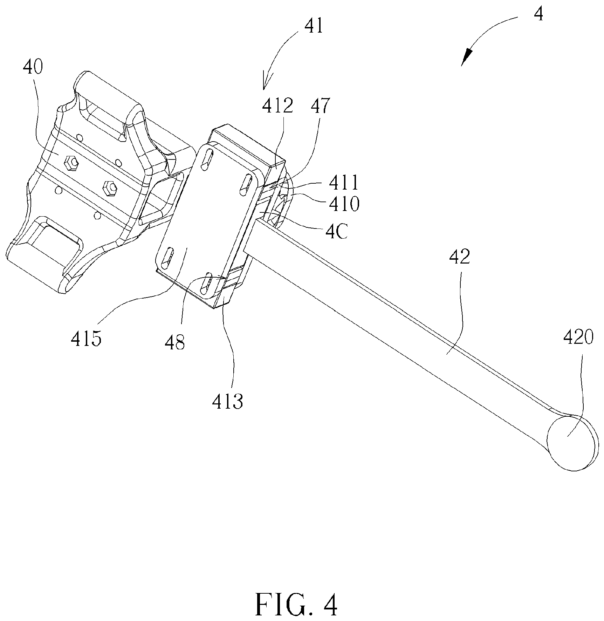

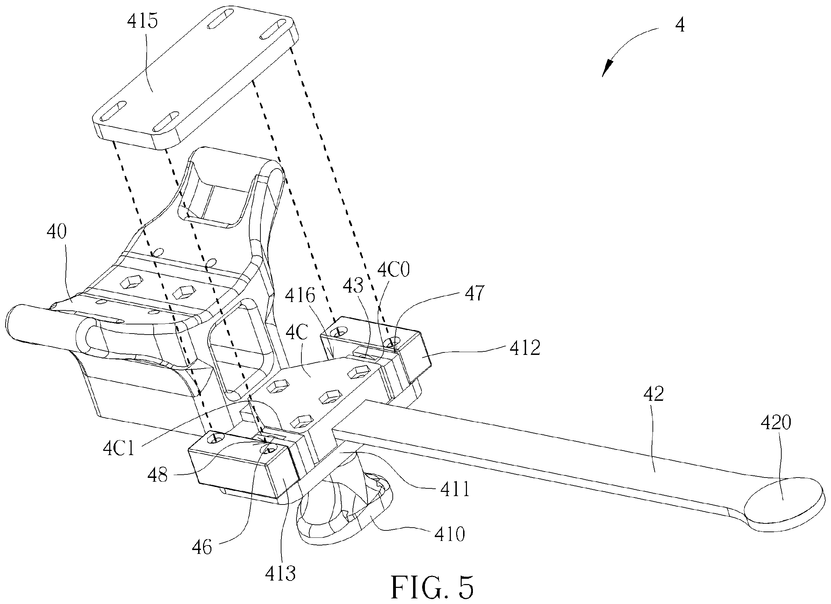

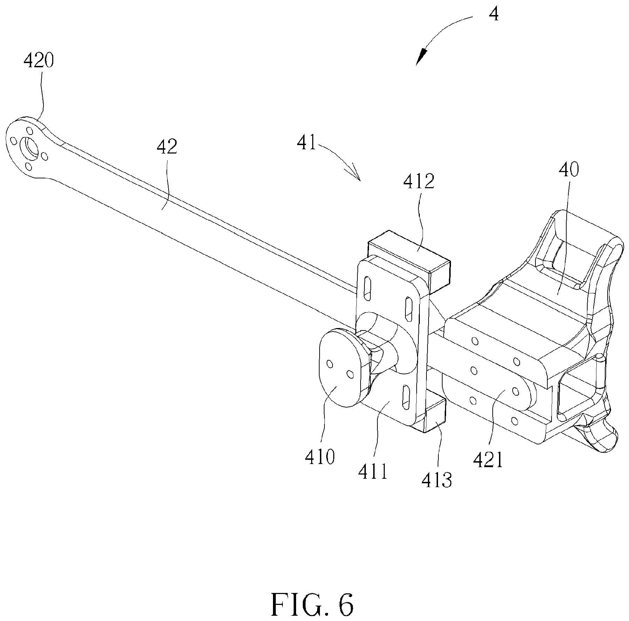

Please refer to FIG. 2 to FIG. 6. FIG. 2 is a diagram illustrating the first exoskeleton member 2, the second exoskeleton member 3, and the sensing module 4. FIG. 3 is a diagram illustrating the first exoskeleton member 2, the second exoskeleton member 3, and the sensing module 4 in another view. FIG. 4 is a diagram illustrating the sensing module 4. FIG. 5 is a partially exploded diagram illustrating the sensing module 4. FIG. 6 is a diagram illustrating the sensing module 4 in another view. As shown in FIG.2 through FIG. 5, the sensing module 4 includes a body attaching member 40, a base module 41, a transmission member 42, a first sensor 43, and an actuating module 44. The body attaching member 40 is attached to a human limb (for example, the forearm) . In practical application, a band can be used to secure the human limb onto the body attaching member 40. The base module 41 is fixed onto the second exoskeleton member 3, and the transmission member 42 is coupled and linked to the body attaching member 40. The first sensor 43 is disposed on a side of the body attaching member 40 and separate from the body attaching member 40, and the actuating module 44 is coupled to the first exoskeleton member 2 and the second exoskeleton member 3.

In the present example, the actuating module 44 includes a first motor driving module 440 and a second motor driving module 441. The first motor driving module 440 is configured to provide a first torque, and the second motor driving module 441 is configured to provide a second torque, wherein the second torque and the first torque are in the same direction. Thereby, the actuating module 44 provides a larger torque output to the first exoskeleton member 2 and the second exoskeleton member 3 through the first motor driving module 440 and the second motor driving module 441. It should be noted that the first motor driving module 440 or the second motor driving module 441 can be omitted in the present invention depending on practical demands. That is, the actuating module 44 can include only one motor driving module based on practical demands. In the present embodiment, the first motor driving module 440 and the second motor driving module 441 are servo motor systems. In addition to the servo motor systems, stepping motors can be used as a power source in the present invention, and it depends on practical demands.

Furthermore, the transmission member 42 includes a pivoting end 420 and a connecting end 421. The pivoting end 420 is pivoted to the first exoskeleton member 2 and the second exoskeleton member 3. The connecting end 421 is connected to the body attaching member 40, and the base module 41 disposed between the pivoting end 420 and the connecting end 421. In the present embodiment, the base module 41 includes a pedestal 410, a carrying platform 411, and a first sensor mounting strip 412. The pedestal 410 is fixed to the second exoskeleton member 3, and the carrying platform 411 is disposed on the pedestal 410. The first sensor mounting strip 412 is disposed on a side of the carrying platform 411, and the first sensor 43 is positioned between the first sensor mounting strip 412 and the transmission member 42. In addition, the sensing module 4 further includes an activating member 4C. The activating member 4C is moveably disposed on the carrying platform 411 and moves with the transmission member 42. That is, the activating member 4C is fixed onto the transmission member 42 in order to move with the transmission member 42. The base module 41 further includes a cover 415, and the cover 415 is fixed onto the first sensor mounting strip 412 and the second sensor mounting strip 413. When the transmission member 42 is configured on the first sensor mounting strip 412 and the second sensor mounting strip 413, the cover 415, the first sensor mounting strip 412, the second sensor mounting strip 413 and the carrying platform 411 can jointly define a containing space 416, so that the activating member 4C is moveably disposed in the containing space 416. Additionally, the activating member 4C has a first side 4C0. The first side 4C0 faces the first sensor mounting strip 412, wherein the first sensor 43 is disposed on the first sensor mounting strip 412 and faces the first side 4C0.

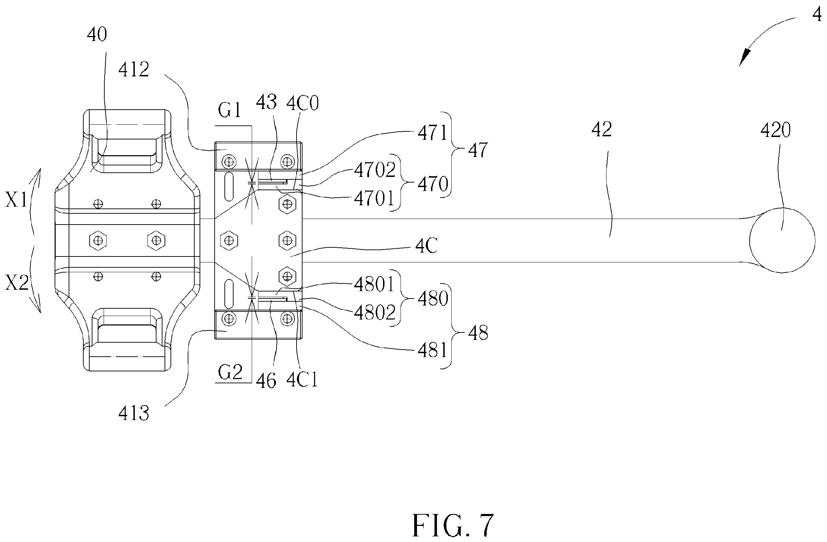

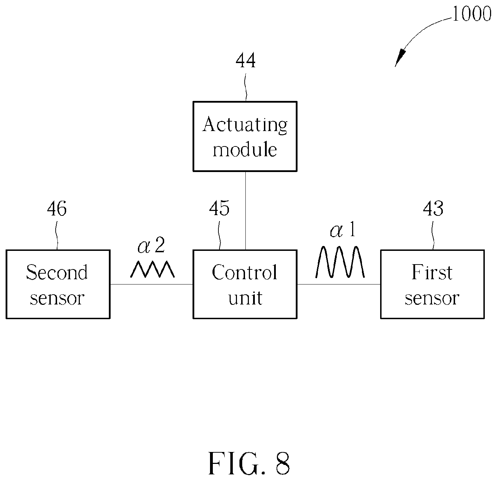

Please refer to FIG. 2 to FIG. 8. FIG. 7 is a front view of the sensing module 4. FIG. 8 is a functional block diagram of the human assistive device 1000. As shown in FIG.2 to FIG. 8, the sensing module 4 further includes a control unit 45, and the control unit 45 is coupled to the first sensor 43 and the actuating module 44. In the present embodiment, the control unit 45 can be a circuit board. In addition, the base module 41 further includes a second sensor mounting strip 413, and the second sensor mounting strip 413 is disposed on another side of the carrying platform 411 and corresponds to the first sensor mounting strip 412. The sensing module 4 further comprises a second sensor 46, and the second sensor 46 corresponds to the first sensor 43 and is separate from the body attaching member 40. The second sensor 46 is positioned between the second sensor mounting strip 413 and the transmission member 42. Furthermore, the activating member 4C has a second side 4C1 opposite to the first side 4C0. The second side 4C1 faces the second sensor mounting strip 413, wherein the second sensor 46 is disposed on the second sensor mounting strip 413 and faces the second side 4C1.

Additionally, the sensing module 4 further includes a first constraining member 47 and a second constraining member 48. The first constraining member 47 is disposed between the first side 4C0 and the first sensor mounting strip 412, and two opposite sides of the first constraining member 47 abut against the first side 4C0 and the first sensor mounting strip 412. The second constraining member 48 is disposed between the second side 4C1 and the second sensor mounting strip 413, and the two sides of the second constraining member 48 abut against the second side 4C1 and the second sensor mounting strip 413. As shown in FIG. 7, the first constraining member 47 includes a first activating member 470 and a first carrying member 471. The first carrying member 471 is disposed on the first sensor mounting strip 412 facing an inner wall of the activating member 4C. The first activating member 470 includes a first flat portion 4701 and a first protrusion 4702, and the first flat portion 4701 is disposed on the first side 4C0 of the activating member 4C. The first protrusion 4702 protrudes out of the first flat portion 4701 and abuts against the first carrying member 471, and the first sensor 43 is disposed on a side of the first carrying member 471 facing the first flat portion 4701. That is, the first sensor 43 is configured on the first sensor mounting strip 412 through the first carrying member 471, thereby placing the first sensor 43 between the activating member 4C and the first sensor mounting strip 412.

As shown in FIG. 7, the second constraining member 48 includes a second activating member 480 and a second carrying member 481, and the second carrying member 481 is disposed on the second sensor mounting strip 413 facing an inner wall of the activating member 4C. The second activating member 480 includes a second flat portion 4801 and a second protrusion 4802, and the second flat portion 4801 is disposed on the second side 4C1 of the activating member 4C. The second protrusion 4802 protrudes from the second flat portion 4801 and abuts against the second carrying member 481, and the second sensor 46 is disposed on a side of the second carrying member 481 facing the second flush portion 4801. That is, the second sensor 46 is configured on the second sensor mounting strip 413 through the second carrying member 481, thereby placing the second sensor 46 between the activating member 4C and the second sensor mounting strip 413.

In addition, the first constraining member 47 and the second constraining member 48 are able to abut the first side 4C0 and the second side 4C1 of the activating member 4C, respectively. Therefore, the first constraining member 47 and the second constraining member 48 can jointly clamp the activating member 4C at an initial position. It is worth noting that the first protrusion 4702 of the first activating member 470 is able to separate the first carrying member 471 and the first flush portion 4701 of the first activating member 470 when the first constraining member 47 and the second constraining member 48 jointly clamp the activating member 4C at the initial position. In such a manner, a first gap G1 can be maintained between the first sensor 43 and the first flush portion 4701, thereby keeping the first flat portion 4701 from contacting the first sensor 43. That is, when the first constraining member 47 and the second constraining member 48 jointly clamp the activating member 4C at the initial position, the first constraining member 47 and the second constraining member 48 do not allow the first sensor 43 to be activated and remain at the zero state. Namely, the second protrusion 4802 of the second activating member 480 can separate the second carrying member 481 and the second flat portion 4801 of the second activating member 480, maintaining a second gap G2 between the second sensor 46 and the second flush portion 4801g. Therefore, the second flush portion 4801 does not contact the second sensor 46. That is, when the first constraining member 47 and the second constraining member 48 jointly clamp the activating member 4C at the initial position, the first constraining member 47 and the second constraining member 48 do not allow the second sensor 46 to be activated and remain at the zero state.

Detailed description on the operating principle of the human assistive device 1000 is provided herein. When the forearm (i.e., the human limb) moves along a first direction X1 relative to the upper arm (that is, a retracted direction for the forearm relative to the upper arm), the body attaching member 40 is driven by the forearm (i.e. the human limb) along the first direction X1. Subsequently, the transmission member 42 and the activating member 4C move along the first direction X1. At this time, the first side 4C0 of the transmission member 42 activates the first sensor 43 disposed on the first sensor mounting strip 412, causing the first sensor 43 to generate a first signal α1 (as shown in FIG. 8). That is, the body attaching member 40 drives the transmission member 42 to activate the first sensor 43 to generate the first signal α1 when the body attaching member 40 is driven by the human limb along the first direction X1. Furthermore, the control unit 45 controls the actuating module 44 to drive the second exoskeleton member 3 to move along the first direction X1 relative to the first exoskeleton member 2 when the control unit 45 receives the first signal α1. In such a way, the human assistive device 1000 can be used to aid the movement of a human limb when a human body is injured, or the human assistive device 1000 can be configured as an exoskeleton device to assist the human body to move loads that exceed the capacity of the human body.

Similarly, when the forearm (i.e., the human limb) moves along a second direction X2 opposite the first direction X1 relative to the upper arm (that is, an expanding direction for the forearm relative to the upper arm), the body attaching member 40 is driven by the forearm (i.e., the human limb) along the second direction X2. Subsequently, the transmission member 42 and the activating member 4C move along the second direction X2. At this time, the second side 4C1 of the transmission member 42 activates the second sensor 46 disposed on the second sensor mounting strip 413, causing the second sensor 46 to generate a second signal α2 (as shown in FIG. 8). That is, the body attaching member 40 drives the transmission member 42 to activate the second sensor 46 to generate the second signal α2 when the body attaching member 40 is driven by the human limb along the second direction X2. Furthermore, the control unit 45 controls the actuating module 44 to drive the second exoskeleton member 3 to move along the second direction X2 relative to the first exoskeleton member 2 when the control unit 45 receives the second signal α2.

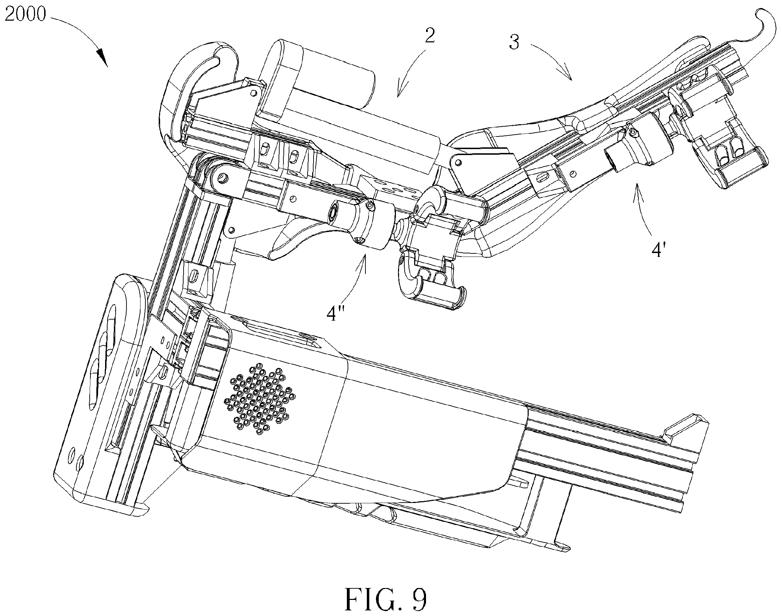

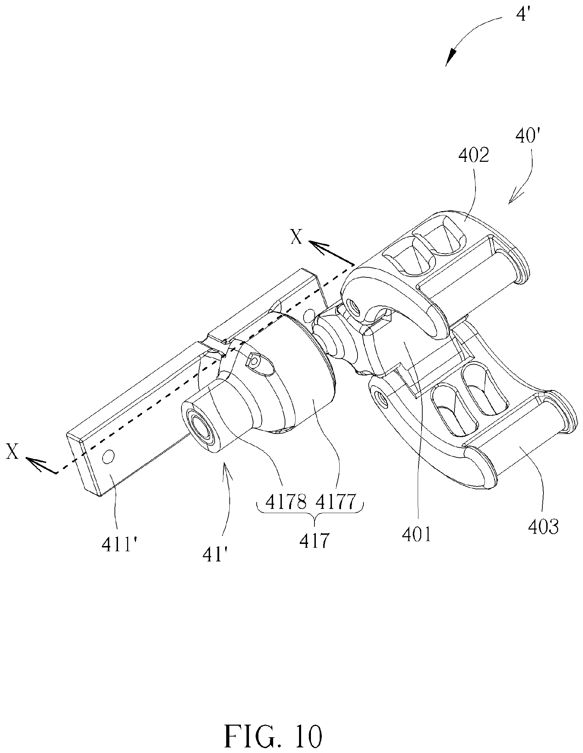

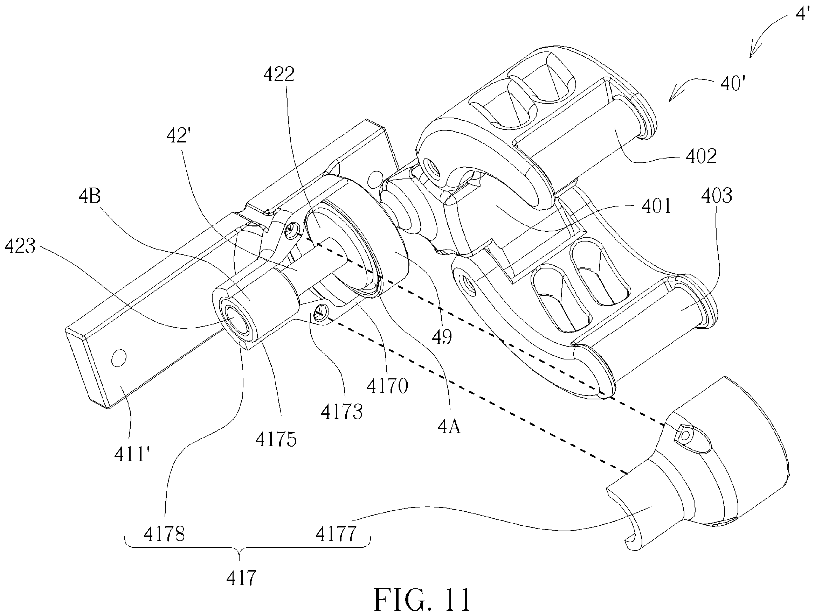

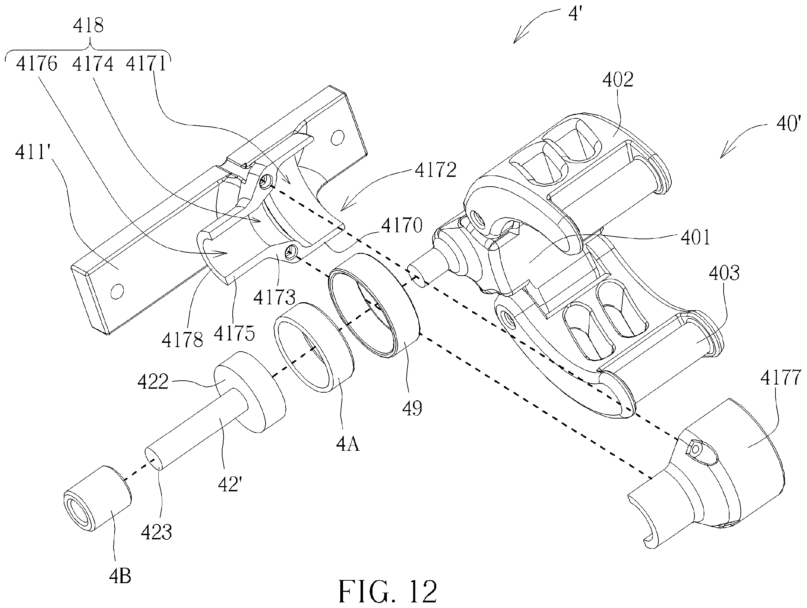

Please refer to FIG. 9. FIG. 9 is a partial diagram illustrating a human assistive device 2000 according to an embodiment of the present invention. As shown in FIG. 9, the human assistive device 2000 includes a torso portion (not shown in figure), a first exoskeleton member 2, a second exoskeleton member 3, and a sensing module 4'. The major difference between the human assistive device 2000 and the aforementioned human assistive device 1000 is in the structural design of the sensing module 4' . Please refer to FIG. 10 to FIG. 12. FIG. 10 is a diagram illustrating the sensing module 4' according to an embodiment of the present invention. FIG. 11 is a partially exploded diagram illustrating the sensing module 4' according to an embodiment of the present invention. FIG. 12 is an exploded diagram illustrating the sensing module 4' according to an embodiment of the present invention. As shown in FIG. 10 to FIG. 12, the sensing module 4' includes a carrying platform 411' and a cup member 417 disposed on a base module 41' . The carrying platform 411' is fixed onto the second exoskeleton member 3, and the cup member 417 is disposed on the second exoskeleton member 3 via the carrying platform 411'. In the present embodiment, the cup member 417 includes an upper cup part 4177 and a lower cup part 4178. The lower cup part 4178 is connected to the carrying platform 411', and the upper cup part 4177 is detachably configured on the lower cup part 4178, facilitating the inner components of the sensing module 4' to be assembled.

Furthermore, the cup member 417 has a top part 4170, a neck part 4173, and a base part 4175. The neck part 4173 connects the top part 4170 and the base part 4175, and the top part 4170, the neck part 4173, and the base part 4175 jointly encase a cup-shaped hollow space 418. A transmission member 42' of the sensing module 4' is disposed in the cup-shaped hollow space 418, and the transmission member 42' has a first connecting end 422 and a second connecting end 423 opposite to each other. The cup-shaped hollow space 418 has an opening 4172 on the top part 4170, and the first connecting end 422 is connected to the body attaching member 40' via the opening 4172. In addition, the top part 4170 surrounds a top spacing 4171, the neck part 4173 surrounds a neck spacing 4174, and the base part 4175 surrounds a base spacing 4176. The top spacing 4171, the neck spacing 4174, and the base spacing 4176 communicate with one another and jointly define the cup-shaped hollow space 418. Moreover, the sensing module 4' further includes a mounting base 49, an activating part 4A, and an initial position-constraining member 4B. The mounting base 49 is disposed on an inner wall of the top part 4170. The first sensor 43 and the second sensor 46 are disposed on the mounting base 49 and correspond to each other. The activating part 4A is fixed on the transmission member 42' and configured to activate the first sensor 43 or the second sensor 46, wherein the mounting base 49 and the activating part 4A are both positioned inside the top spacing 4171. In the present embodiment, the mounting base 49 can be a ring-shaped member and made of elastic materials (e.g., rubber).

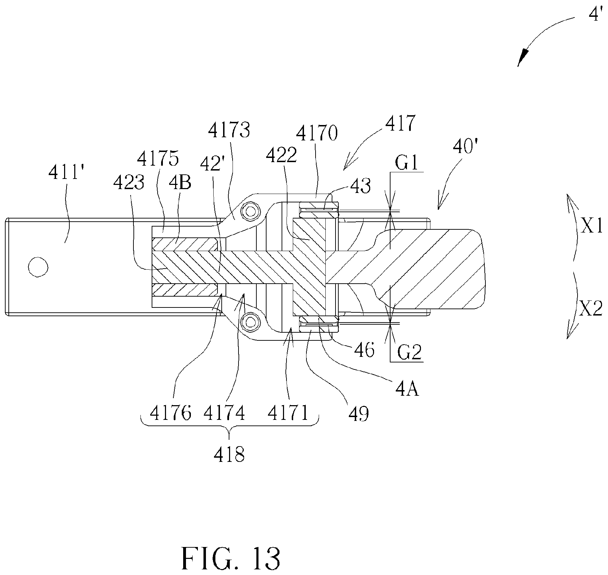

In the present embodiment, the initial position-constraining member 4B is disposed inside the base spacing 4176. Please refer to FIG. 10 to FIG. 13. FIG. 13 is a cross-section diagram of the sensing module 4' along section line X-X as shown in FIG. 10. As shown in FIG. 10 to FIG. 13, the initial position-constraining member 4B holds the second connecting end 423 of the transmission member 42' so that when the transmission member 42' is at an initial status as shown in FIG. 10 and FIG. 13, a gap (that is, the first gap G1 and the first gap G2) is allowed to be present between the activating part 4A and the mounting base 49. The principle of the sensing module 4' is identical to that of the sensing module 4, and related descriptions are omitted for simplicity. It is worth noting that when the upper arm and the forearm of the human limb are fitted with two sensing modules 4", 4' , as shown in FIG. 9, respectively, executing a controlled motion in which the forearm swings relative to the upper arm (leading to the arm being raised slowly) results in the sensing module 4" generating smooth signals shown in FIG. 15, which are different from the unstable signals shown in FIG. 14, even if the upper arm muscle swells during the aforementioned swinging motion.

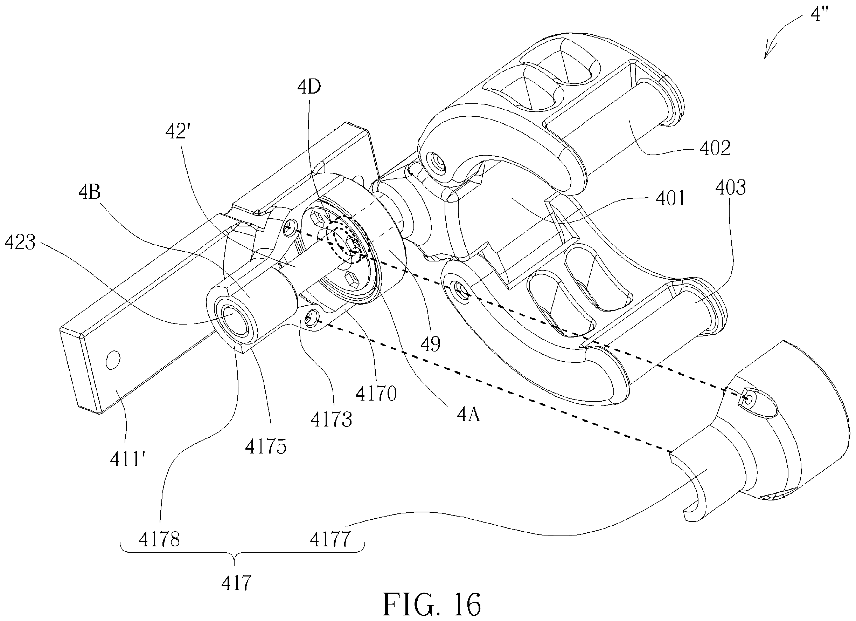

Please refer to FIG. 16. FIG. 16 is a diagram illustrating a sensing module 4" according to another embodiment of the present invention. As shown in FIG. 16, the major difference between the sensing module 4" and the aforementioned sensing module 4' is that the sensing module 4" further includes a rotating bearing 4D, and a transmission member 42' is rotatably disposed in the rotating bearing 4D. Therefore, the sensing module 4" is able to eliminate the noise resulting from shear forces generated by the pressure sensor inside the sensing module 4" when the upper arm rotates relative to the human torso. The activating part 4A can freely rotate through the rotating bearing 4D relative to the transmission member 42'. Therefore, the transmission member 42' can engage (and not affect) the pressure sensor through the activating part 4A without generating unnecessary noise resulting from shear forces. That is, the rotating bearing 4D can eliminate the noise in the sensing module 4" resulting from shear forces as the human limb rotates. The operating principle of the sensing module 4" is identical to that of the sensing module 4, and related descriptions are omitted for simplicity.

Compared to the prior art, the sensing module of the present invention is attached to the human limb using the body attaching member, and the sensor (s) of the sensing module is/are configured on the sensor mounting strip or the cup member, wherein the sensor mounting strip or the cup member is separate from the body attaching member. Furthermore, the sensing module of the present invention connects the transmission member to the body attaching member, allowing the human limb to drive the body attaching member during movement and resulting in the transmission member activating the sensor to generate a signal. Therefore, the sensor of the present invention can be free from the effects of muscle swelling and skin friction during human limb movement due to the separation of the sensor from the human limb. Subsequently, the actuating module can drive the exoskeleton member to move more precisely.

## Claims

1. A human assistive device (2000) comprising a sensing module (4', 4") capable of reducing noise generated by muscle swelling and skin friction during human body movement, the human assistive device (2000) comprising a torso portion (1), a first exoskeleton member (2), and a second exoskeleton member (3), one end of the first exoskeleton member (2) being pivoted to the torso portion (1), the second exoskeleton member (3) being pivoted to another end of the first exoskeleton member (2), the sensing module (4' , 4") being positioned on at least one of the first exoskeleton member (2) and the second exoskeleton member (3), the sensing module (4', 4") comprising:
a body attaching member (40') configured to be attached to a human limb;
a base module (41') fixed onto at least one of the first exoskeleton member (2) and the second exoskeleton member (3) and comprising:
a carrying platform (411') fixed onto the second exoskeleton member (3); and
a cup member (417) disposed on the carrying platform (411'), the cup member (417) encasing a cup-shaped hollow space (418), the cup-shaped hollow space (418) having an opening (4172);
a transmission member (42') disposed in the cup-shaped hollow space (418) and comprising a first connecting end (422) and a second connecting end (423);
a first sensor (43); a second sensor (46);
a mounting base (49) disposed on an inner wall of the cup member (417), the first sensor (43) and the second sensor (46) being disposed on the mounting base (49);
an activating part (4A) fixed onto the transmission member (42'), the activating part (4A) being configured to activate the first sensor (43) and the second sensor (46) on the mounting base (49) ;
an initial position-constraining member (4B) disposed inside the cup-shaped hollow space (418); and
the second sensor (46) corresponding to the first sensor (43), the first sensor (43) and the second sensor (46) being separate from the body attaching member (40');
wherein the first connecting end (422) of the transmission member (42') is being connected to the body attaching member (40') via the opening (4172);
wherein the body attaching member (40') drives the transmission member (42') and the activating part (4A) to activate the first sensor (43) to generate a first signal (α1) when the body attaching member (40') is driven along a first direction (X1) by the human limb;
wherein the initial position-constraining member (4B) holds the second connecting end (423) and allows a gap to be present between the activating part (4A) and the mounting base (49) when the transmission member (42') is in an initial status;
wherein the body attaching member (40') drives the activating part (4A) to activate the second sensor (46) to generate a second signal (α2) when the body attaching member (40') is driven by the human limb along a second direction (X2) opposite to the first direction (X1).

2. The human assistive device (2000) of claim 1, further **characterized by**:
a rotating bearing (4D) disposed inside the activating part (4A), the transmission member (42') being rotatably disposed inside the rotating bearing (4D).

3. The human assistive device (2000) of claims 1-2, further **characterized by**:
an actuating module (44) coupled to the first exoskeleton member (2) and the second exoskeleton member (3); and
a control unit (45) coupled to the first sensor (43) and the actuating module (44), the control unit (45) controlling the actuating module (44) to drive the second exoskeleton member (3) to move along the first direction relative to the first exoskeleton member (2) when the control unit (45) receives the first signal (α1).

4. The human assistive device (2000) of claim 3, further **characterized in that** the control unit (45) controls the actuating module (44) to drive the second exoskeleton member (3) to move along the second direction (X2) relative to the first exoskeleton member (2) when the control unit (45) receives the second signal (α2).

5. The human assistive device (2000) of claims 3-4, **characterized in that** the actuating module (44) comprises a first motor driving module (440), and the first motor driving module (440) is configured to provide a first torque.

6. The human assistive device (2000) of any of claims 3-5, **characterized in that** the actuating module (44) further comprises a second motor driving module (441), and the second motor driving module (441) is configured to provide a second torque, the second torque and the first torque being in the same direction.

## Patentansprüche

1. Hilfsvorrichtung für Menschen (2000), welche ein Sensormodul (4', 4") umfasst, das geeignet ist, Geräusche zu reduzieren, die durch Muskelschwellung und Hautreibung während der menschlichen Körperbewegung erzeugt werden, worin die Hilfsvorrichtung für Menschen (2000) einen Rumpfabschnitt (1), ein erstes Exoskelettelement (2) und ein zweites Exoskelettelement (3) umfasst, worin ein Ende des ersten Exoskelettelementes (2) an dem Rumpfabschnitt (1) schwenkbar angeordnet ist, das zweite Exoskelettelement (3) an einem anderen Ende des ersten Exoskelettelementes (2) schwenkbar angeordnet ist, das Sensormodul (4', 4") auf mindestens einem von dem ersten Exoskelettelement (2) und dem zweiten Exoskelettelement (3) angeordnet ist, worin das Sensormodul (4', 4") umfasst:
ein Körperbefestigungselement (40'), das ausgestaltet ist, an einer menschlichen Gliedmaße befestigt zu werden;
ein Basismodul (41'), das an mindestens einem von dem ersten Exoskelettelement (2) und dem zweiten Exoskelettelement (3) befestigt ist und welches umfasst:
eine Trageplattform (411'), die an dem zweiten Exoskelettelement (3) befestigt ist; und
ein Schalenelement (417), das auf der Trageplattform (411') angeordnet ist, worin das Schalenelement (417) einen schalenförmigen Hohlraum (418) umschließt, und der schalenförmige Hohlraum (418) eine Öffnung (4172) aufweist;
ein Übertragungselement (42'), das in dem schalenförmigen Hohlraum (418) angeordnet ist und ein erstes Verbindungsende (422) und ein zweites Verbindungsende (423) umfasst;
einen ersten Sensor (43);
einen zweiten Sensor (46);
eine Befestigungsbasis (49), die an einer Innenwand des Schalenelementes (417) angeordnet ist, worin der erste Sensor (43) und der zweite Sensor (46) an der Befestigungsbasis (49) angeordnet sind;
ein Aktivierungselement (4A), das an dem Übertragungselement (42') befestigt ist, worin das Aktivierungselement (4A) ausgestaltet ist, den ersten Sensor (43) und den zweiten Sensor (46) auf der Befestigungsbasis (49) zu aktivieren;
ein Anfangspositions-Beschränkungselement (4B), das innerhalb des schalenförmigen Hohlraums (418) angeordnet ist; und
worin der zweite Sensor (46) dem ersten Sensor (43) entspricht, und der erste Sensor (43) und der zweite Sensor (46) von dem Körperbefestigungselement (40') getrennt sind;
worin das erste Verbindungsende (422) des Übertragungselements (42') über die Öffnung (4172) mit dem Körperbefestigungselement (40') verbunden ist;
wobei das Körperbefestigungselement (40') das Übertragungselement (42') und den aktivierenden Teil (4A) antreibt, den ersten Sensor (43) zu aktivieren, um ein erstes Signal (α1) zu erzeugen, wenn das Körperbefestigungselement (40') durch die menschliche Extremität entlang einer ersten Richtung (X1) angetrieben wird;
wobei das Anfangspositions-Beschränkungselement (4B) das zweite Verbindungsende (423) hält und ermöglicht, dass ein Spalt zwischen dem aktivierenden Teil (4A) und der Befestigungsbasis (49) vorliegt, wenn sich das Übertragungselement (42') in einem Anfangszustand befindet;
wobei das Körperbefestigungselement (40') das aktivierende Teil (4A) antreibt, den zweiten Sensor (46) zu aktivieren, um ein zweites Signal (α2) zu erzeugen, wenn das Körperbefestigungselement (40') von der menschlichen Gliedmaße entlang einer zweiten Richtung (X2) entgegengesetzt zur ersten Richtung (X1) angetrieben wird.

2. Hilfsvorrichtung für Menschen (2000) nach Anspruch 1, ferner **gekennzeichnet durch**:
ein Drehlager (4D), das innerhalb des aktivierenden Teils (4A) angeordnet ist, worin das Übertragungselement (42') innerhalb des Drehlagers (4D) drehbar angeordnet ist.

3. Hilfsvorrichtung für Menschen (2000) nach Anspruch 1 bis 2, ferner **gekennzeichnet durch**:
ein Betätigungsmodul (44), das mit dem ersten Exoskelettelement (2) und dem zweiten Exoskelettelement (3) gekoppelt ist; und
eine Steuereinheit (45), die mit dem ersten Sensor (43) und dem Betätigungsmodul (44) gekoppelt ist, worin die Steuereinheit (45) das Betätigungsmodul (44) steuert, um das zweite Exoskelettelement (3) anzutreiben, sich entlang der ersten Richtung relativ zu dem ersten Exoskelettelement (2) zu bewegen, wenn die Steuereinheit (45) das erste Signal (α1) empfängt.

4. Hilfsvorrichtung für Menschen (2000) nach Anspruch 3, ferner **dadurch gekennzeichnet, dass** die Steuereinheit (45) das Betätigungsmodul (44) steuert, um das zweite Exoskelettelement (3) anzutreiben, sich entlang der zweiten Richtung (X2) relativ zu dem ersten Exoskelettelement (2) zu bewegen, wenn die Steuereinheit (45) das zweite Signal (α2) empfängt.

5. Hilfsvorrichtung für Menschen (2000) nach Anspruch 3-4, **dadurch gekennzeichnet, dass** das Betätigungsmodul (44) ein erstes Motorantriebsmodul (440) umfasst, und das erste Motorantriebsmodul (440) ausgestaltet ist, ein erstes Drehmoment bereitzustellen.

6. Hilfsvorrichtung für Menschen (2000) nach einem der Ansprüche 3-5, **dadurch gekennzeichnet, dass** das Betätigungsmodul (44) ferner ein zweites Motorantriebsmodul (441) umfasst, und das zweite Motorantriebsmodul (441) ausgestaltet ist, ein zweites Drehmoment bereitzustellen, wobei das zweite Drehmoment und das erste Drehmoment in dieselbe Richtung weisen.

## Revendications

1. Dispositif d'assistance à un être humain (2000) comprenant un module de détection (4', 4") pouvant réduire le bruit généré par le gonflement musculaire et le frottement de la peau pendant un mouvement du corps humain, le dispositif d'assistance à un être humain (2000) comprenant une partie de torse (1), un premier élément d'exosquelette (2) et un deuxième élément d'exosquelette (3), une extrémité du premier élément d'exosquelette (2) étant pivotée vers la partie de torse (1), le deuxième élément d'exosquelette (3) étant pivoté vers une autre extrémité du premier élément d'exosquelette (2), le module de détection (4', 4") étant positionné sur au moins un élément parmi le premier élément d'exosquelette (2) et le deuxième élément d'exosquelette (3), le module de détection (4', 4") comprenant:
un élément de fixation au corps (40') conçu pour être fixé à un membre humain;
un module de base (41') fixé sur au moins un élément parmi le premier élément d'exosquelette (2) et le deuxième élément d'exosquelette (3) et comprenant:
une plateforme support (411') fixée sur le deuxième élément d'exosquelette (3); et
un élément de coupelle (417) disposé sur la plateforme support (411'), l'élément de coupelle (417) enfermant un espace creux en forme de coupelle (418), l'espace creux en forme de coupelle (418) présentant une ouverture (4172);
un élément de transmission (42') disposé dans l'espace creux en forme de coupelle (418) et comprenant une première extrémité de liaison (422) et une deuxième extrémité de liaison (423);
un premier capteur (43);
un deuxième capteur (46);
une base de montage (49) disposée sur une paroi interne de l'élément de coupelle (417), le premier capteur (43) et le deuxième capteur (46) étant disposés sur la base de montage (49);
une partie d'activation (4A) fixée sur l'élément de transmission (42'), la partie d'activation (4A) étant conçue pour activer le premier capteur (43) et le deuxième capteur (46) sur la base de montage (49);
un élément de contrainte de position initiale (4B) disposé à l'intérieur de l'espace creux en forme de coupelle (418); et
le deuxième capteur (46) correspondant au premier capteur (43), le premier capteur (43) et le deuxième capteur (46) étant séparés de l'élément de fixation au corps (40');
la première extrémité de liaison (422) de l'élément de transmission (42') étant reliée à l'élément de fixation au corps (40') par l'intermédiaire de l'ouverture (4172);
l'élément de fixation au corps (40') entraînant l'élément de transmission (42') et la partie d'activation (4A) pour activer le premier capteur (43) en vue de générer un premier signal (α1) lorsque l'élément de fixation au corps (40') est entraîné dans un premier sens (X1) par le membre humain;
l'élément de contrainte de position initiale (4B) maintenant la deuxième extrémité de liaison (423) et permettant la présence d'un espace entre la partie d'activation (4A) et la base de montage (49) lorsque l'élément de transmission (42') est dans un état initial;
l'élément de fixation au corps (40') entraînant la partie d'activation (4A) pour activer le deuxième capteur (46) en vue de générer un deuxième signal (α2) lorsque l'élément de fixation au corps (40') est entraîné par le membre humain dans un deuxième sens (X2) opposé au premier sens (X1).

2. Dispositif d'assistance à un être humain (2000) selon la revendication 1, **caractérisé en outre par**:
un palier rotatif (4D) disposé à l'intérieur de la partie d'activation (4A), l'élément de transmission (42') étant disposé de manière rotative à l'intérieur du palier rotatif (4D).

3. Dispositif d'assistance à un être humain (2000) selon les revendications 1-2, **caractérisé en outre par**:
un module d'actionnement (44) accouplé au premier élément d'exosquelette (2) et au deuxième élément d'exosquelette (3); et
une unité de commande (45) couplée au premier capteur (43) et au module d'actionnement (44), l'unité de commande (45) commandant le module d'actionnement (44) pour entraîner le déplacement du deuxième élément d'exosquelette (3) dans le premier sens par rapport au premier élément d'exosquelette (2) lorsque l'unité de commande (45) reçoit le premier signal (α1).

4. Dispositif d'assistance à un être humain (2000) selon la revendication 3, **caractérisé en outre en ce que** l'unité de commande (45) commande le module d'actionnement (44) pour entraîner le déplacement du deuxième élément d'exosquelette (3) dans le deuxième sens (X2) par rapport au premier élément d'exosquelette (2) lorsque l'unité de commande (45) reçoit le deuxième signal (α2).

5. Dispositif d'assistance à un être humain (2000) selon les revendications 3-4, **caractérisé en ce que** le module d'actionnement (44) comprend un premier module d'entraînement de moteur (440) et le premier module d'entraînement de moteur (440) est conçu pour fournir un premier couple.

6. Dispositif d'assistance à un être humain (2000) selon l'une quelconque des revendications 3-5, **caractérisé en ce que** le module d'actionnement (44) comprend en outre un deuxième module d'entraînement de moteur (441) et le deuxième module d'entraînement de moteur (441) est conçu pour fournir un deuxième couple, le deuxième couple et le premier couple étant dans le même sens.
